# EUROPEAN PATENT SPECIFICATION

(11) **EP 3 213 640 B1**
(45) Date of publication and mention of the grant of the patent: **21.07.2021**
(21) Application number: 15855610.0
(22) Date of filing: 29.09.2015
(51) Int. Cl.: A23D 9/007, A23D 9/00, A23L 33/115

(54) **LONG-CHAIN POLYUNSATURATED FATTY-ACID-CONTAINING FAT AND FOOD CONTAINING SAME**
FETT MIT LANGKETTIGER, MEHRFACH UNGESÄTTIGTER FETTSÄURE UND LEBENSMITTEL DAMIT
MATIÈRE GRASSE CONTENANT DES ACIDES GRAS POLYINSATURÉS À LONGUE CHAÎNE ET ALIMENTS EN COMPRENANT

(30) Priority: 30.10.2014 JP 2014221627
(43) Date of publication of application: 06.09.2017
(73) Proprietor: Fuji Oil Holdings Inc., Osaka 598-8540 (JP)
(72) Inventor: KATO,Masaharu, Tsukubamirai-shi Ibaraki 300-2436 (JP); MORIKAWA,Miwako, Tsukubamirai-shi Ibaraki 300-2436 (JP); NOGUCHI,Yoshie, Tsukubamirai-shi Ibaraki 300-2436 (JP)
(74) Representative: Boult Wade Tennant LLP
(86) International application number: PCT/JP2015/077424
(87) International publication number: WO 2016/067804

(56) References cited:
- EP-A2- 0 705 539
- WO-A1-2005/005585
- WO-A1-2008/103939
- WO-A1-2013/066373
- WO-A1-2013/147131
- JP-A- H0 353 869
- JP-A- H09 263 784
- JP-A- 2001 346 517
- US-A1- 2004 151 757
- Ed Kane: "Dietary antioxidants are important for pets", DVM360 Magazine, 1 February 2004 (2004-02-01), Retrieved from the Internet: URL:http://veterinarynews.dvm360.com/dieta ry-antioxidants-are-important-pets?id=&sk= &date=&pageID=2 [retrieved on 2019-04-04]
- Evellyn C?mara Grilo ET AL: "Alpha-tocopherol and gamma-tocopherol concentration in vegetable oils", Food Science and Technology (Campinas), vol. 34, no. 2, 1 June 2014 (2014-06-01), pages 379-385, XP055245185, DOI: 10.1590/S0101-20612014005000031

## Description

### Technical Field

The present invention relates to a long-chain polyunsaturated fatty acid-containing fat. In more detail, the present invention relates to a long-chain polyunsaturated fatty acid-containing fat, and a food containing the same which implement improvement of oxidation stability, which is required from a food containing a long-chain polyunsaturated fatty acid-containing fat.

### Background Art

In recent years, it has been known that fats containing a long-chain polyunsaturated fatty acid have a physiological action, and these fats are also widely used for usage for addition into health foods and feed from health consciousness. Especially, n-3 long-chain polyunsaturated fatty acids containing 5 to 6 double bonds, such as eicosapentaenoic acid (EPA) and docosahexaenoic acid (DHA), have been reported to have a large number of actions such as decrease in neutral fat and cholesterol in the blood, alleviation of symptoms of rheumatoid arthritis, prevention of formation of a thrombus and anti-allergenic action, and have come to be recognized as a functional food by many consumers. Herein, the fat containing a long-chain polyunsaturated fatty acid is written as a long-chain polyunsaturated fatty acid-containing fat.

A long-chain polyunsaturated fatty acid-containing fat has very worse oxidation stability as compared with that of edible oils such as soybean oil and rapeseed oil, and is easily oxidized by oxygen, heat, light and the like, to generate off-taste and off-flavor like fish by oxidation and produce a peroxide substance of which adverse effect on the health is concerned. For utilizing such long-chain polyunsaturated fatty acid-containing fat having remarkably inferior oxidation stability, various methods aimed at blocking contact with oxygen in air are disclosed. Patent Documents 1 to 8 are disclosed as methods of making an emulsion, and Patent Documents 9 to 10 are disclosed as methods of powderization and capsulation. However, usage of the emulsion is limited because the fat becomes turbid and contains moisture. In addition, there is a problem that usage of powderized or capsulated fat is also limited as with the case of the emulsion. According to such technical background, utilization of a conventional long-chain polyunsaturated fatty acid-containing fat is limited to the application range such as a health food covered with a capsule and a canned food. A process of manufacturing capsules is complicated and the productivity is low. Deterioration of capsulated products has already progressed in the course of the step, and most of the oil inside the capsules has oxidative rancidity. In addition, there is a problem that the kinds of capsules which can be used as a food, feed or the like are limited, in order to prevent destruction of preserved capsules.

Patent Document 11 discloses a method of utilizing a food oil composition for cooking, by mixing a fat containing DHA, of which oxidation stability is inferior, at a ratio of 30% or more with other oil. However, the amount of combined DHA is as extremely small as 0.01 to 0.5% in such a method, and greatly inferior to intake the amount regarded as effective for health.

Purified long-chain polyunsaturated fatty acid-containing fat does not have oxidative rancidity or a peroxide substance. However, once oxidation occurs, it is known that the reaction occurs in a chain reaction, and that oxidative rancidity and production of a peroxide substance rapidly occur. For example, when purified fish oil is used in a factory, various cares such as enclosing nitrogen gas after use of the oil, sealing a can containing the oil and the gas, and freezing the can are needed for handling the fat. Thus, the utilization is spontaneously limited. There have been many attempts to improve oxidation stability of a long-chain polyunsaturated fatty acid-containing fat by addition of a wide variety of oxidation inhibitors or a mixture thereof. However, those attempts have not arrived at sufficient results so far, and it cannot be said that development of a food containing a long-chain polyunsaturated fatty acid-containing fat is sufficiently progressing, contrary to requirement of the market.

Conventionally, addition of an antioxidant is generally carried out for improving oxidation stability of an edible fat. More specifically, utilization of tocopherol and ascorbic acid is examined. For example, Patent Document 12 is disclosed as a method of adding tocopherol, ascorbic acid and tea extract; Patent Document 13 is disclosed as a method of adding ascorbic acid or a derivative thereof and other organic acid or a derivative thereof; and Patent Document 2 is disclosed as a method of emulsifying and adding an aqueous ascorbic acid solution. However, those methods are not satisfactory as a method for improving oxidation stability of a long-chain polyunsaturated fatty acid-containing fat, and further excellent method for preventing oxidation is desired.

In addition, manufacturers of additives consider improvement of solubility of a water-soluble antioxidative substance to oil by using a large amount of emulsifier, and sell products. However, combination of a large amount of emulsifier causes an adverse effect on flavor and is not preferable, and problems of oxidative deterioration and putrid odor have not been solved yet.

As a method for improving oxidation stability of a long-chain polyunsaturated fatty acid-containing fat, Patent Document 14 discloses a method characterized by subjecting fish oil treated with silica gel to vacuum steam deodorization in the presence of rosemary and sage extract and adding palmitic acid-ascorbic acid ester or Mixed Tocopherol. A fat having high oxidation stability without the problem of the aroma characteristic of rosemary and sage is proposed by the method. However, the best product obtained by the method has rancidity stability only increased three times at 100°C in the combination of 3000 ppm of an antioxidative substance regarding the oxidation stability of the long-chain polyunsaturated fatty acid, and thus the oxidation stability is still low. Although Patent Document 15 discloses a method of adding catechin before purification, it cannot be said that improvement of oxidation stability indicated by peroxide value is sufficient even in such a method.

In addition, as a method for reducing odor associated with deterioration, Patent Document 16 is proposed as a method of adding dairy flavor, and Patent Document 17 is proposed as a method of masking fish odor by adding ginger flavor. However, a masking treatment which does not suppress oxidation cannot fundamentally solve the problem because an oxidation of fat produces peroxide which is adverse for a human body.

Relation between oxidization stability of a fat and the number of double bonds contained in the fatty acid is strong, and it is known that, as the number of double bonds contained in the fatty acid is large, oxidization stability remarkably decreases. Then, Patent Documents 18 to 19 show examples of using interesterification technique of fish oil with a fat, such as solid fat and hydrogenated fat having a few double bonds in the fatty acid and high oxidation stability, and changing the structure of a long-chain polyunsaturated fatty acid-containing fat to improve stability and apply the obtained fat to a food. Patent Documents 20 to 22 disclose a method for improving oxidation stability by applying slightly hydrogenating treatment to fish oil.

As described above, it has been conventionally thought that it is important to decrease the number of double bonds in a molecule of triglyceride or to decrease the number of double bonds composing a long-chain polyunsaturated fatty acid as one of the methods of maintaining flavor stability of a long-chain polyunsaturated fatty acid such as EPA and DHA. Examples of such methods include an interesterification with a fat containing many saturated fatty acids having high oxidation stability and a slightly hydrogenating treatment of fish oil itself.

However, the fat becomes solid at a normal temperature when these procedures are carried out, and thus foods and usages for which the fat can be used are limited. Moreover, EPA and DHA contents are decreased when fish oil itself is subjected to a slightly hydrogenating treatment. Thus, there has been the possibility that the physiological activities are also remarkably decreased.

In order to increase oxidation stability of a long-chain polyunsaturated fatty acid-containing fat while maintaining the state of liquid at a normal temperature, attempts to increase oxidation stability of a polyunsaturated fatty acid by mixing with a triglyceride which contains a lot of monounsaturated fatty acids having high oxidation stability next to saturated acid (for example, high-oleic sunflower oil and olive oil) are also made mainly in on-market products. In order to improve oxidation stability of α-linolenic acid, which is n-3 polyunsaturated fatty acid, a method of adding a high-oleic fat is reported in Patent Document 23. Patent Document 24 also shows an example of combining 30 to 60% palm olein oil or 10 to 30% high-oleic sunflower oil with an antioxidative substance for improving thermal stability of α-linolenic acid.

However, the polyunsaturated fatty acid-containing fat to which the high-oleic fat is combined has low oxidation stability, and is difficult to be handled in the same manner as a general fat. In addition, Patent Documents 23 and 24 do not describe DHA and EPA having remarkably lower oxidation stability than that of α-linolenic acid.

Patent Document 25 shows a method of suppressing fish odor for a long time while maintaining a long-chain polyunsaturated fatty acid such as DHA and EPA in the state of liquid. Concretely, a fat composition having a fatty acid ratio of 3 to 9 parts by weight of oleic acid, 5 to 15 parts by weight of linoleic acid and 0.1 to 1.5 parts by weight of α-linolenic acid on the basis of 1 part by weight of a long-chain polyunsaturated fatty acid such as DHA and EPA is disclosed. And, it is shown that the ratio of oleic acid and linoleic acid of the combined fat is important for improvement of oxidation stability of the long-chain polyunsaturated fatty acid.

### Prior Art Documents

### Patent Documents

Patent Document 1: JP 2011-255373 A
Patent Document 2: JP H07-107938 A
Patent Document 3: JP 2005-529728 A
Patent Document 4: JP H07-313055 A
Patent Document 5: JP H08-154576 A
Patent Document 6: JP H08-205771 A
Patent Document 7: JP H08-154577 A
Patent Document 8: JP H06-49479 A
Patent Document 9: JP H07-305088 A
Patent Document 10: JP H09-87656 A
Patent Document 11: JP 2013-81477 A
Patent Document 12: JP H09-111237 A
Patent Document 13: JP H09-235584 A
Patent Document 14: JP 2000-144168 A
Patent Document 15: JP 2005-124439 A
Patent Document 16: JP H06-68 A
Patent Document 17: JP H06-189717 A
Patent Document 18: JP H06-287593 A
Patent Document 19: JP H07-308153 A
Patent Document 20: JP H07-216383 A
Patent Document 21: JP H07-264985 A
Patent Document 22: JP H07-268385 A
Patent Document 23: JP 2007-159589 A
Patent Document 24: JP H07-83677 B
Patent Document 25: JP 3884465 B1

EP 0705539 relates to a food based on milk products, intended for pregnant and lactating women, which food is specifically supplemented with docosahexaenoic acid and eicosapentaenoic acid and nutrient fibres in an amount accounting for more than 10% of the total mono- and disaccharide fraction. US 2004/0151757 relates to a method for supplementing the diet of a subject with diabetes mellitus comprising administering to the subject medium-chain triglycerides (MCT) for optimization as to food physiology of the fatty acid spectrum in a dietary foodstuff. The use of MCT and a dietary foodstuff containing these MCT for particular medicinal purposes for supplementary monitored nutrition/dietary treatment of adults and teenagers with diabetes mellitus is described. WO 2008/103939 discloses compositions and methods to promote bone development, enhance neurologic development, enhance cognitive and motor skills, promote a healthy body composition, and/or reduce the fat of a growing animal.

### Summary of Invention

### Problems to be Solved by Invention

As described above, a long-chain polyunsaturated fatty acid-containing fat is a material attracting attention due to the effectiveness of the long-chain polyunsaturated fatty acid on a human body. However, it has inferior oxidation stability as compared with that of a general edible fat. Thus, products processed into a capsule, an emulsion and the like for the purpose of blocking oxygen and utilized predominate in the prior art, and there is a problem that the usage applications are limited.

An object of the present invention is to sufficiently suppress oxidation of a long-chain polyunsaturated fatty acid-containing fat; to provide a long-chain polyunsaturated fatty acid-containing fat which has good flavor and can be distributed in a state of liquid as is the case with a general edible oil such as soybean oil and rapeseed oil; and to widely expand the usage to the food field, in order to meet the above-mentioned demands.

### Means for Solving Problems

As described above, Patent Document 25 shows that the ratio of oleic acid and linoleic acid of the combined fat is important for improvement of oxidation stability of a long-chain polyunsaturated fatty acid. It has been thought that this increases oxidation stability while keeping the state of liquid and has been a highly versatile technique. However, it has been found that increase in peroxide value (POV) which is important for use for a food cannot be sufficiently suppressed. Furthermore, it has been suggested that it is difficult to make a long-chain polyunsaturated fatty acid highly contained and to more improve oxidation stability only by adjusting the fatty acid composition.

The present inventors have extensively studied for the above problem. As a result, they have found that it is effective to have a specific linoleic acid content and a specific tocopherol/tocotrienol content, to complete the present invention.

That is, the present invention is:
(1) a long-chain polyunsaturated fatty acid-containing fat comprising 1 to 5 parts by weight of linoleic acid and 0.001 part by weight to 0.05 part by weight of total amount of the following components (A), on the basis of 1 part by weight of the content of a long-chain polyunsaturated fatty acid having 5 or more double bonds in the constituent fatty acid composition, where
   components (A): β-tocopherol, γ-tocopherol, δ-tocopherol, β-tocotrienol, γ-tocotrienol and δ-tocotrienol, and wherein the content of the long-chain polyunsaturated fatty acid having 5 or more double bonds in the constituent fatty acid composition is 7 to 30% by weight;
(2) the long-chain polyunsaturated fatty acid-containing fat of (1), where the long-chain polyunsaturated fatty acid having 5 or more double bonds is DHA and/or EPA;
(3) the long-chain polyunsaturated fatty acid-containing fat of (1) or (2),
   further comprising an oxidation inhibitor;
4) the long-chain polyunsaturated fatty acid-containing fat of (3), where the oxidation inhibitor is at least one agent selected from the group consisting of ascorbic acid, ascorbic acid derivative, tea extract, gallic acid, gallic acid derivative and lecithin;
(5) the long-chain polyunsaturated fatty acid-containing fat of any one of (1) to (4), which is in a state of liquid at 20°C;
(6) a food including the long-chain polyunsaturated fatty acid-containing fat of any one of (1) to (5);
(7) a long-chain polyunsaturated fatty acid-containing fat of (1) wherein a peroxide value after three days obtained from a preservation test of the long-chain polyunsaturated fatty acid-containing fat described below is 65 meq/kg or less: (Method of preservation test of long-chain polyunsaturated fatty acid-containing fat)
   - putting 30 g of uniformly mixed fat into a 100-ml Erlenmeyer flask;
   - letting the Erlenmeyer flask opened to contact with air; and
   - stirring the Erlenmeyer flask in a thermostatic shaking incubator (rotation number: 100 rpm, set at 60°C) with heating;
(8) a method for suppressing increase in peroxide value of a long-chain polyunsaturated fatty acid-containing fat, comprising making a content of linoleic acid to 1 to 5 parts by weight and the total amount of the components (A) to 0.001 part by weight to 0.05 part by weight, on the basis of 1 part by weight of the content of the long-chain polyunsaturated fatty acid having 5 or more double bonds in the constituent fatty acid composition wherein
   components (A): β-tocopherol, γ-tocopherol, δ-tocopherol, β-tocotrienol, γ-tocotrienol and δ-tocotrienol, and wherein the content of the long-chain polyunsaturated fatty acid having 5 or more double bonds in the constituent fatty acid composition is 7 to 30% by weight;
(9) use of the long-chain polyunsaturated fatty acid-containing fat of any of (1) to (5) or (7) in a food; and
(10) a method for suppressing increase in peroxide value of a food including using the long-chain polyunsaturated fatty acid-containing fat of any one of (1) to (5) or (7).

### Effects of Invention

According to the present invention, a long-chain polyunsaturated fatty acid-containing fat of which oxidation stability is improved and which has good flavor and may be distributed in a state of liquid may be obtained. Therefore, a long-chain polyunsaturated fatty acid-containing fat, where generation of reversion odor and unpleasant odor, and peroxide which adversely acts on health is suppressed for a long period of time, and having high health advantage may be used more inexpensively than conventional products. And, the oxidation stability may be further improved by adding a relatively small amount of antioxidative substance. In addition, the long-chain polyunsaturated fatty acid-containing fat of the present invention may be used without limiting the range of use to the state of liquid. More specifically, the long-chain polyunsaturated fatty acid-containing fat of the present invention enables to obtain an emulsion or to process the emulsion into a form of powder, a capsule or the like. The long-chain polyunsaturated fatty acid-containing fat of the present invention may be utilized for wider usages encompassing the range of use of conventional long-chain polyunsaturated fatty acid-containing fats.

### Mode for Carrying out Invention

The kind of long-chain polyunsaturated fatty acid-containing fat to be used in the present invention is not specifically limited, so long as the long-chain polyunsaturated fatty acid-containing fat is composed of a long-chain polyunsaturated fatty acid having 5 or more double bonds and the number of carbons of 20 or more, and the long-chain polyunsaturated fatty acid-containing fat is edible. The long-chain polyunsaturated fatty acid-containing fat may include various animal and vegetable oils such as fish oil and vegetable oils, fats obtained from microorganisms and algae. In terms of purity and cost, fish oil, and long-chain polyunsaturated fatty acid-containing fat derived from algae are preferable. At least one of such long-chain polyunsaturated fatty acid-containing fat may be used, and the long-chain polyunsaturated fatty acid-containing fat of the present invention may include two or more kinds of long-chain polyunsaturated fatty acid-containing fats.

A ratio of the long-chain polyunsaturated fatty acid having 5 or more double bonds to the fatty acids in the total fat is preferably 1.5% by weight to 60% by weight, more preferably 1.5% by weight to 30% by weight, and, in terms of an resulting effect of improvement of oxidation stability, further preferably 1.5% by weight to 20% by weight, and most preferably 7.0% by weight to 20% by weight. The kind of the fatty acid having 5 or more double bonds is not specifically limited, for example, docosahexaenoic acid (DHA; C22:6), eicosapentaenoic acid (EPA; C20:5) and docosapentaenoic acid (DPA; C22:5) may be used. In terms of high concentration in fish oil and the long-chain polyunsaturated fatty acid-containing fat derived from algae, DHA and/or EPA is preferable.

The long-chain polyunsaturated fatty acid-containing fat of the present invention is a long-chain polyunsaturated fatty acid-containing fat which may be distributed in the state of liquid as is the case with a general edible oil such as soybean oil and rapeseed oil, and preferably characterized by being in the state of liquid at 20°C. In addition, the long-chain polyunsaturated fatty acid-containing fat of the present invention is also characterized by having low moisture, and the moisture content is preferably 5% by weight or less, and more preferably 2% by weight or less.

As the linoleic acid-containing fat used in the present invention, any linoleic acid-containing fat known to a skilled person in the art may be used. Examples of preferable linoleic acid-containing fat include vegetable fat such as soybean oil, corn oil, rapeseed oil, palm olein, and safflower oil. In terms of having tocopherol composition suitable for the present invention, it is preferable to use soybean oil and/or corn oil as a linoleic acid-containing fat. Two or more kinds of such fats may be mixed and used.

The long-chain polyunsaturated fatty acid-containing fat of the present invention includes 1 to 5 parts by weight of linoleic acid on the basis of 1 part by weight of the content of the long-chain polyunsaturated fatty acid having 5 or more double bonds in the constituent fatty acid. More preferably, it is preferable that the content of the long-chain polyunsaturated fatty acid having 5 or more double bonds in the constituent fatty acid composition is 7 to 30% by weight.
Components (A): β-tocopherol, γ-tocopherol, δ-tocopherol, β-tocotrienol, γ-tocotrienol, and δ-tocotrienol.

Tocopherol and/or tocotrienol component may be separately added to the long-chain polyunsaturated fatty acid-containing fat of the present invention as the components (A). However, it is preferable that the components be originally contained in the oil without further adding the components because requirement of consumers in terms of a price and reduction of additives may be satisfied. Regarding the antioxidants which may be used as tocopherol and/or tocotrienol component, two or more kinds of antioxidants containing tocopherol and/or tocotrienol may be appropriately mixed and used.

An oxidation inhibitor may be added to the long-chain polyunsaturated fatty acid-containing fat of the present invention. Examples of the oxidation inhibitor include oil-soluble antioxidant, water-soluble antioxidative substance such as myrica extract, rosemary extract, polyphenol derived from grape juice or tea, ascorbic acid and derivative thereof, gallic acid and derivative thereof. Such oxidation inhibitors may be used in combination. A preferable oxidation inhibitor is at least one kind selected from ascorbic acid, ascorbic acid derivative, tea extract, gallic acid, gallic acid derivative and lecithin.

In addition, an additive, which may be used for an edible fat, such as flavor, coloring agent and silicone may be used for the long-chain polyunsaturated fatty acid-containing fat of the present invention, as necessary.

The long-chain polyunsaturated fatty acid-containing fat of the present invention enables to remarkably suppress increase in peroxide value (POV). In utilization of the long-chain polyunsaturated fatty acid-containing fat of the present invention, it is known that POV increase and deterioration of flavor are correlated. In the present invention, evaluation by POV has been performed in order to visualize the difference which is difficult to distinguish only by flavor evaluation. In addition, it is known that peroxide not only adversely affects flavor but also adversely affects health, and it is essential to suppress increase in POV upon use for food. The fat of which increase in POV is suppressed has good flavor, and a food having little change in flavor may be obtained by utilizing the fat of the present invention of which increase in POV is suppressed.

As one aspect of the long-chain polyunsaturated fatty acid-containing fat of the present invention, a long-chain polyunsaturated fatty acid-containing fat suitable for the range of content of a long-chain polyunsaturated fatty acid having 5 or more double bonds in the constituent fatty acid composition may be obtained, by making: the content of a long-chain polyunsaturated fatty acid having 5 or more double bonds in the constituent fatty acid composition to 7% by weight or more and 30% by weight or less; linoleic acid to 1 to 5 parts by weight and the total amount of the components (A) to 0.001 part by weight to 0.05 part by weight on the basis of 1 part by weight of the content of the long-chain polyunsaturated fatty acid having 5 or more double bonds in the constituent fatty acid composition; and giving the peroxide value after three days obtained from the preservation test of the long-chain polyunsaturated fatty acid-containing fat described below of 65 meq/kg or less. And, the obtained long-chain polyunsaturated fatty acid-containing fat has good flavor even upon use for a food and may suppress increase in peroxide value. (Method of preservation test of long-chain polyunsaturated fatty acid-containing fat)
- putting 30 g of uniformly mixed fat into a 100-ml Erlenmeyer flask;
- letting the Erlenmeyer flask opened to contact with air; and
- stirring the Erlenmeyer flask in a thermostatic shaking incubator (rotation number: 100 rpm, set at 60°C) with heating.

As another aspect of the long-chain polyunsaturated fatty acid-containing fat of the present disclosure, a long-chain polyunsaturated fatty acid-containing fat suitable for the range of content of a long-chain polyunsaturated fatty acid having 5 or more double bonds in the constituent fatty acid composition may be obtained, by making the content of a long-chain polyunsaturated fatty acid having 5 or more double bonds in the constituent fatty acid composition to 1.5% by weight or more and less than 7% by weight; linoleic acid to 5 to 35 parts by weight and the total amount of the components (A) as 0.001 part by weight to 0.05 part by weight on the basis of 1 part by weight of the content of the long-chain polyunsaturated fatty acid; and giving the peroxide value after three days obtained from the preservation test of the long-chain polyunsaturated fatty acid-containing fat of 40 meq/kg or less. And, the obtained long-chain polyunsaturated fatty acid-containing fat has good flavor even upon use for a food, and may suppress increase in peroxide value.

The preservation test of the long-chain polyunsaturated fatty acid-containing fat may be performed by an arbitrary method as necessary. For example, the test may be performed on an arbitrary amount of fat depending on the form and capacity of the container, or the thermostatic machine in which the preservation test is performed. Regarding the method of contact with air, pressurized air may be blown into the container containing the fat. The rotation number and temperature condition of the thermostatic shaking incubator may be appropriately adjusted. Although the time required for the preservation test is long, the fat may be left standing still and kept without stirring and/or heating in the thermostatic shaking incubator.

The long-chain polyunsaturated fatty acid-containing fat of the present invention may obtain good evaluation in flavor evaluation by sensory evaluation, and may be used for various foods for which it has been difficult to use conventional long-chain polyunsaturated fatty acid-containing fats. Examples of preferable usages include dressing, shortening, margarine, sandwich cream, chocolate, frozen dessert, deep-frying oil, spray oil, mayonnaise, margarine, cookies, capsules, powder, other processed fat food.

### Examples

Examples of the present invention will be explained in more detail herein below. In the examples, both of % and part mean weight basis.

### <Method of flavor evaluation of long-chain polyunsaturated fatty acid-containing fat>

Evaluation was carried out by 8 panelists on a four level scale by the following basis:
○; no sensible odor
Δ; slightly sensible odor
×; oxidative rancidity
××; irritating odor

### (Example 1 to Example 5, Comparative Example 1 to Comparative Example 5)

By using purified fish oil and various purified vegetable oils having the fatty acid composition and tocopherol/tocotrienol shown in Table 1, the fat was uniformly mixed at the ratio described in Tables 2, 4 and 5. POV of the mixed fat was 0. Thirty grams of the mixed fat were put into a 100-ml Erlenmeyer flask and stirred in a thermostatic shaking incubator (rotation number: 100 rpm, set at 60°C) with the flask opened, and sampling was carried out over time, to analyze POV (meq/kg). Flavor evaluation was carried out in terms of Table 2. The results are shown in Table 3. Additionally, in Example 4, tocopherol composition was appropriately adjusted by adding 0.1 part by weight of Mixed Tocopherol (manufactured by RIKEN VITAMIN Co., Ltd.) on the basis of the fat combination of Comparative Example 1.

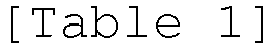

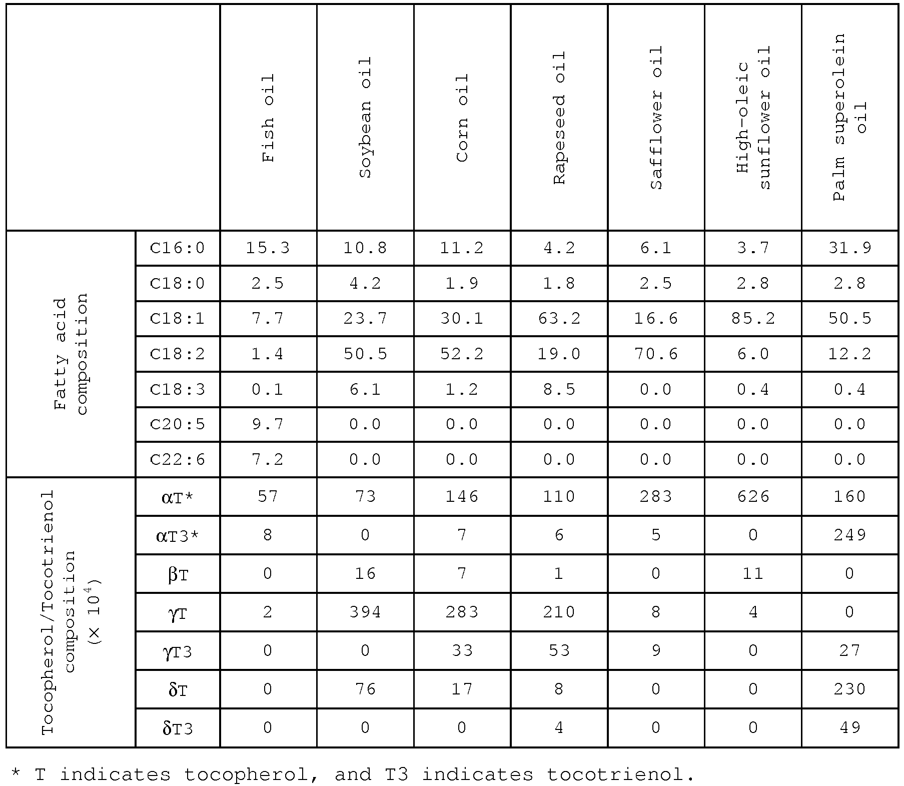

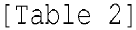

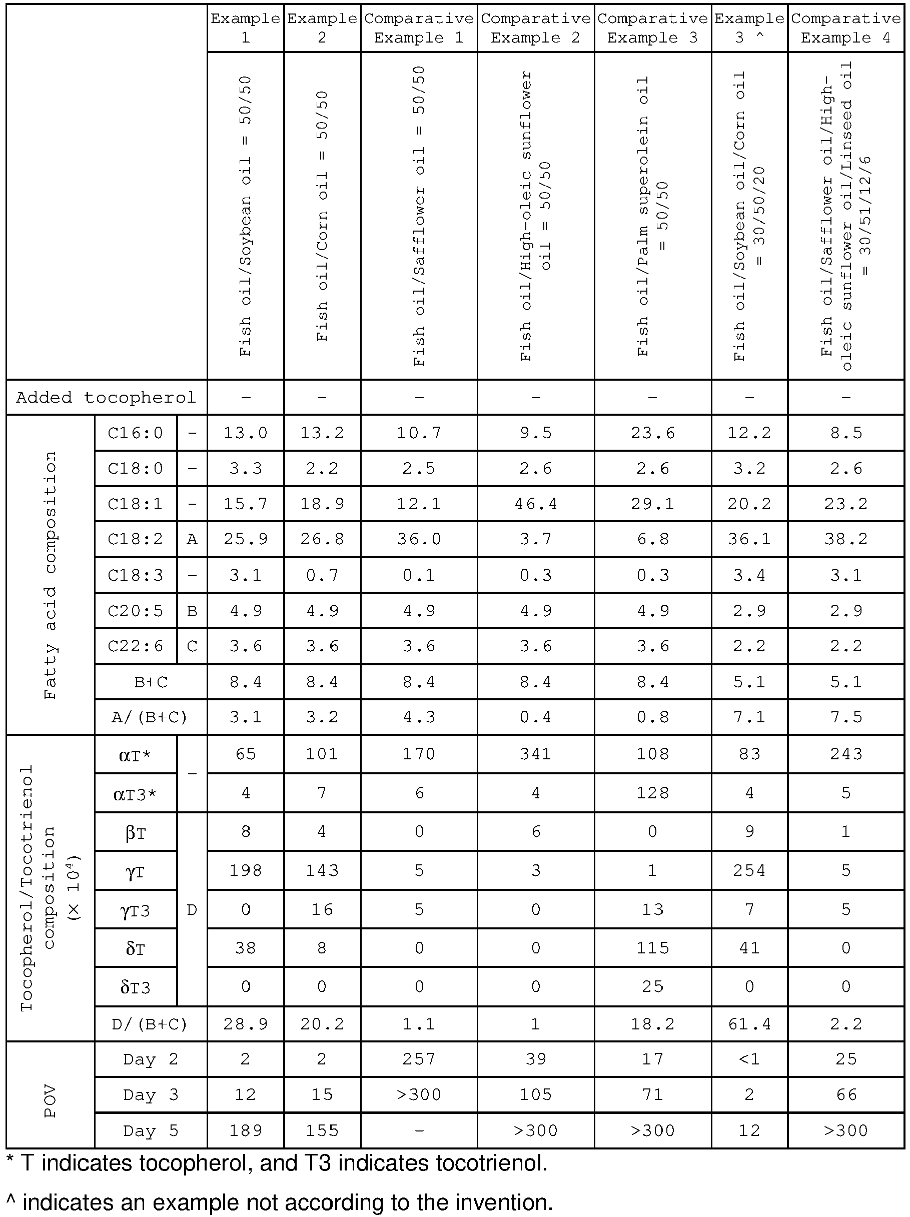

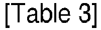

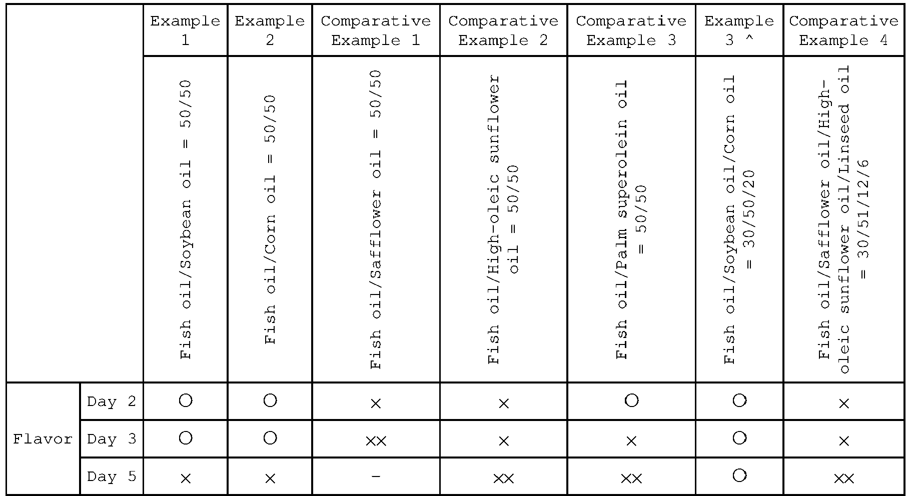

Fatty acid composition, tocopherol/tocotrienol and POV values are shown in Table 2. It became clear that increase in POV was remarkably high in a case of where the fatty acid composition ratio (A/(B+C)) and the tocopherol/tocotrienol ratio (D/(B+C)) to the DHA+EPA content (B+C) did not satisfy the conditions of the present invention, in comparison of the DHA/EPA content in Examples 1 to 2 and Comparative Examples 1 to 3. In addition, from the comparison of Comparative Example 1 and Examples 1 and 2, it was shown that not only the fatty acid composition ratio (A/(B+C)) but also the tocopherol/tocotrienol ratio (D/(B+C)) was important for suppression of increase in POV value. Furthermore, it became clear that (Comparative Example 1), which satisfied the claimed scope of Patent Document 26 in which effect of suppressing fish odor was regarded as high, did not suppress increase in POV, and that increase in POV was suppressed by using a fat of which fatty acid composition ratio (A/(B+C)) and tocopherol/tocotrienol ratio (D/(B+C)) to DHA+EPA content (B+C) satisfied the conditions of the present invention.

Additionally, better flavor evaluation was obtained in Examples as compared with that of Comparative Examples.

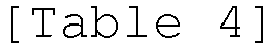

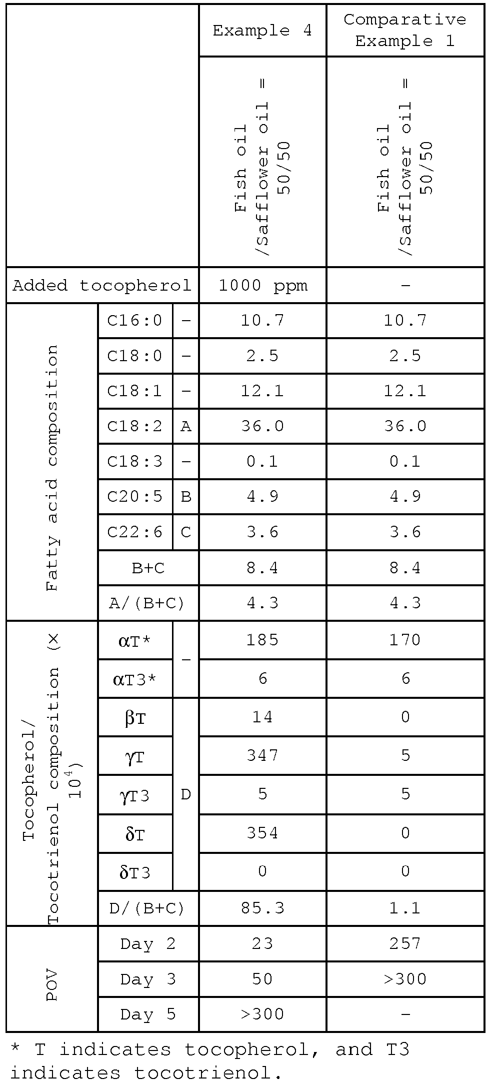

In Example 4, in which Mixed Tocopherol was additionally added to appropriately adjust the tocopherol/tocotrienol ratio (D/ (B+C)) to DHA+EPA content (B+C), increase in POV was suppressed as compared with Comparative Example 1, which had an equivalent fat combination.

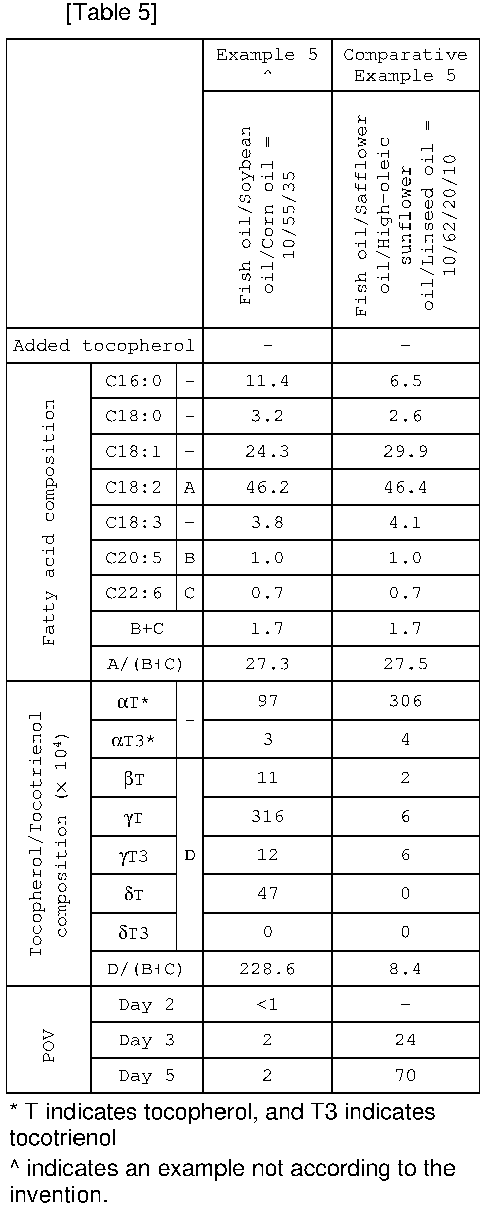

It became clear that increase in POV was small and oxidation stability was high in Example 5 in which the fatty acid composition ratio (A/(B+C)) and the tocopherol/tocotrienol ratio (D/(B+C)) to the DHA+EPA content (B+C) were appropriately adjusted, even under conditions of low DHA/EPA content.

### (Example 6 to Example 13 and Comparative Example 6 to Comparative Example 11)

By using purified tuna oil and various purified vegetable oils having the fatty acid composition and tocopherol/tocotrienol shown in Table 6, the fat was uniformly mixed at the ratio described in Tables 7 and 9. POV of the mixed fat was 0. Thirty grams of the mixed fat were put into a 100-ml Erlenmeyer flask and stirred in a thermostatic shaking incubator (rotation number: 100 rpm, set at 60°C) with the flask opened, and sampling was carried out over time, to analyze POV (meq/kg). Flavor evaluation was carried out in terms of Table 7. The results are shown in Table 8. Additionally, in Example 13, 0.05 part by weight of tea polyphenol-containing composition (manufactured by Taiyo Kagaku Co., Ltd., product name: Sunphenon 90S) was added to the combination described in Example 12. Additionally, the long-chain polyunsaturated fatty acid-containing fat of Example 13 was prepared by preparing an aqueous solution to which 50% by weight tea polyphenol-containing composition was dissolved, adding 0.1 part by weight of the 50% by weight aqueous tea polyphenol solution and 0.1 part by weight of emulsifier (manufactured by RIKEN VITAMIN Co., Ltd., POEM PR-100) to 99.8 parts by weight of oil, and stirring the mixture by a homomixer (TK ROBO MIX, manufactured by Tokusyu Kika Kogyo Co., Ltd.) at 10000 rpm × 5 minutes.

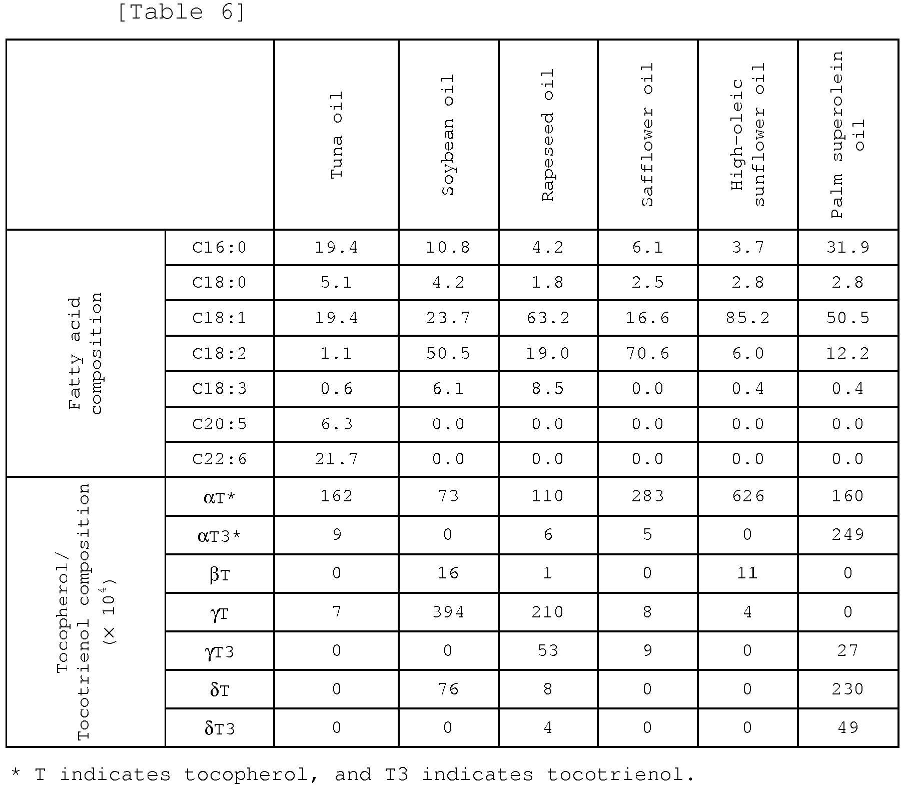

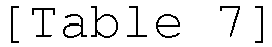

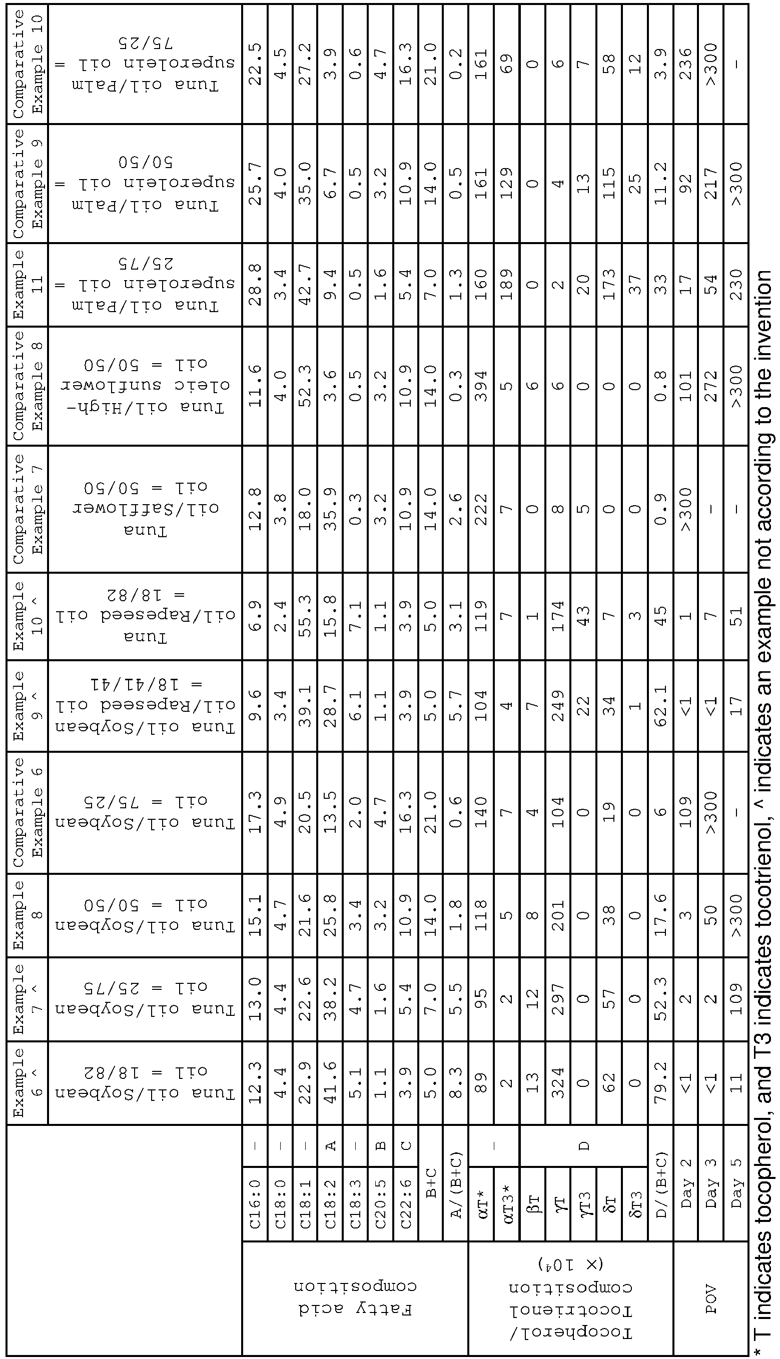

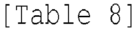

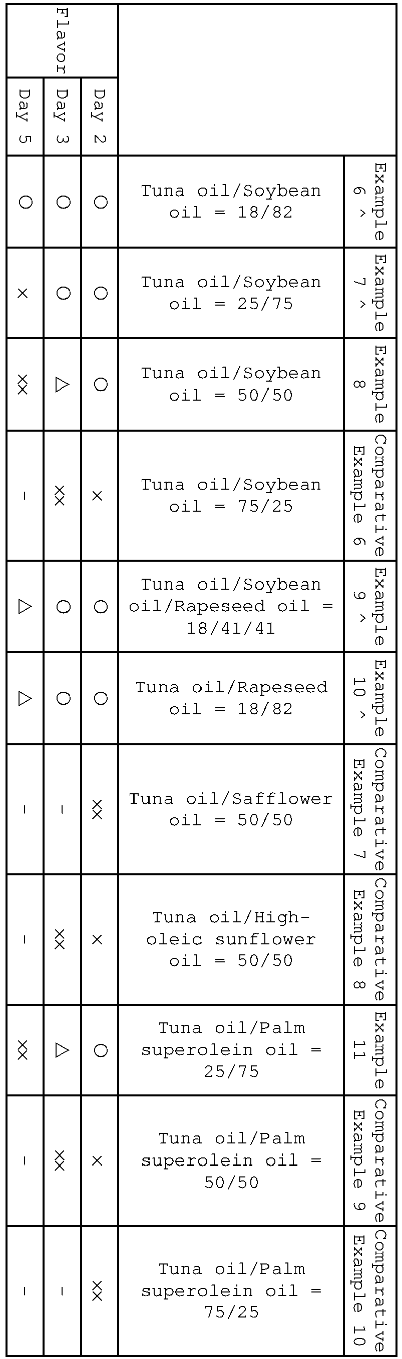

In tuna oil, there was also a tendency that increase in POV was suppressed and that oxidation stability of the fat was high in Examples in which the fatty acid composition ratio (A/(B+C)) and the tocopherol/tocotrienol ratio (D/(B+C)) to the DHA+EPA content (B+C) were appropriately adjusted, as is the case with the test on fish oil. For example, while DHA/EPA content was same in Example 8 and Comparative Examples 7 and 9, increase in POV over time was small and oxidation stability was remarkably high in Example 8, which corresponds to the conditions of the present invention.

Additionally, good flavor evaluation was obtained in Examples as compared with that in Comparative Examples.

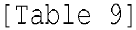

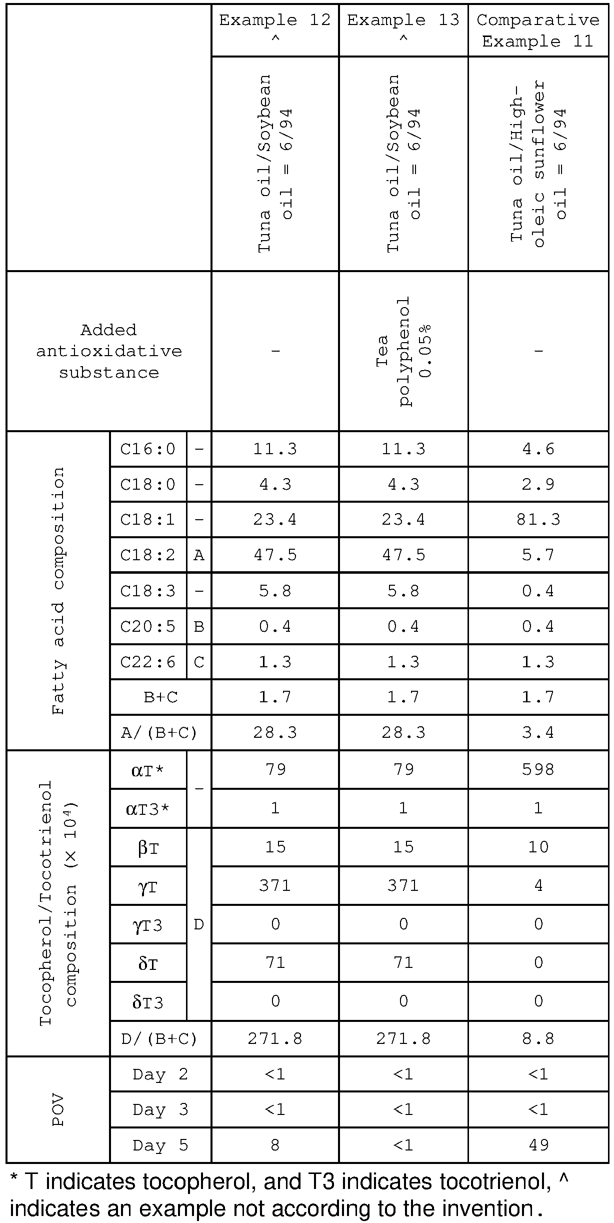

It became clear that increase in POV was small and that oxidation stability was high in Examples in which the fatty acid composition ratio (A/(B+C)) and the tocopherol/tocotrienol ratio (D/(B+C)) to the DHA+EPA content (B+C) were appropriately adjusted, even in Examples 12 and 13 in which tocopherol+tocotrienol total content was smaller than that of Comparative Example 11, under the conditions of low DHA/EPA content. In addition, from Examples 12 and 13, it also became clear that oxidation stability was further increased by adding an oxidation inhibitor.

### (Example 14, Example 15, Comparative Example 12 and Comparative Example 13)

By using purified algae oil and various purified vegetable oils having the fatty acid composition and tocopherol/tocotrienol shown in Table 10, the fat was uniformly mixed at the ratio described in Tables 11 and 12. POV of the mixed fat was 0. Thirty grams of the mixed fat were put into a 100-ml Erlenmeyer flask and stirred in a thermostatic shaking incubator (rotation number: 100 rpm, set at 60°C) with the flask opened, and sampling was carried out over time, to analyze POV (meq/kg).

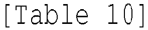

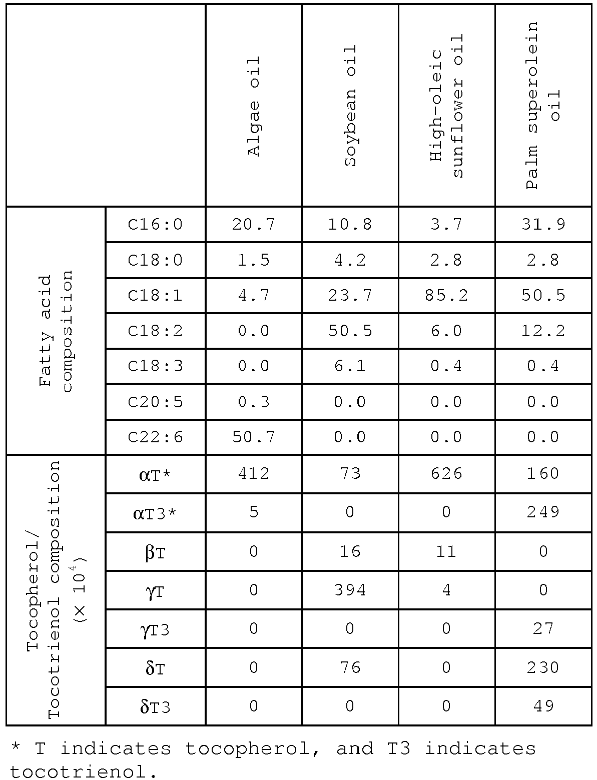

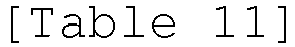

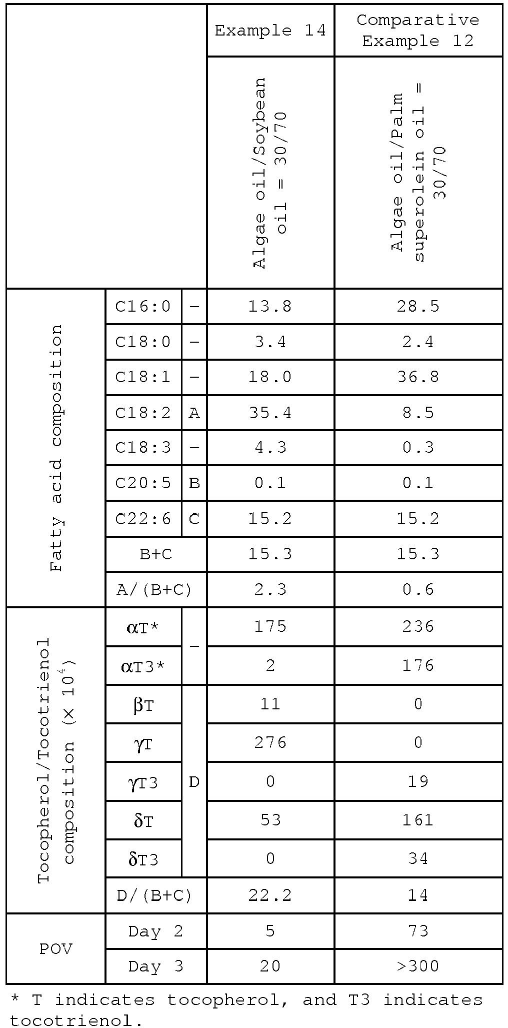

While the contents of DHA and EPA were same in Example 14 and Comparative Example 12, increase in POV was small and oxidation stability was high in Example 14 in which the fatty acid composition ratio (A/(B+C)) and the tocopherol/tocotrienol ratio (D/(B+C)) to the DHA+EPA content (B+C) were appropriately adjusted.

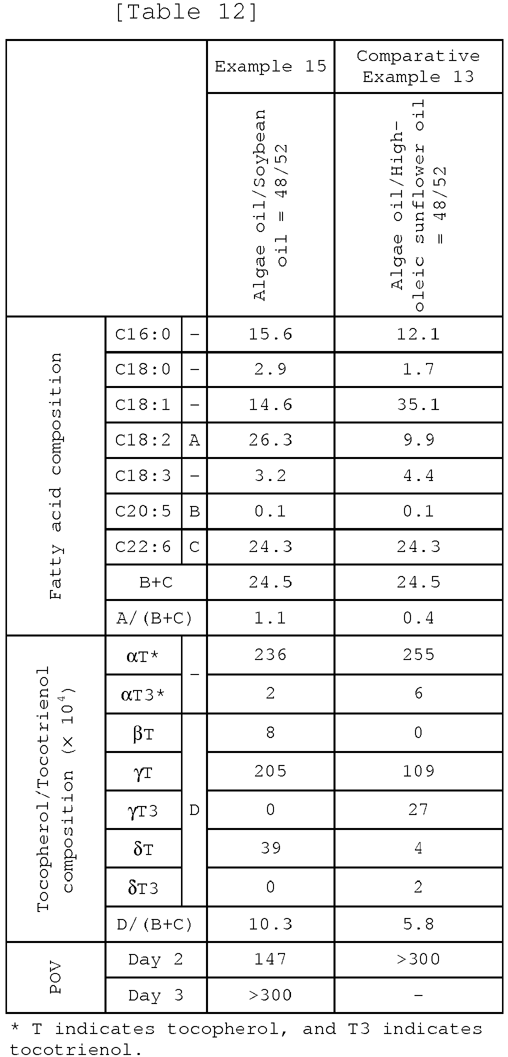

While the contents of DHA and EPA were same in Example 15 and Comparative Example 13, increase in POV was small and oxidation stability was high in Example 15 in which the fatty acid composition ratio (A/(B+C)) and the tocopherol/tocotrienol ratio (D/(B+C)) to the DHA+EPA content (B+C) were appropriately adjusted.

### (Example 16 and Example 17)

By using purified tuna oil and various purified vegetable oils having the fatty acid composition and tocopherol/tocotrienol shown in Tables 1 and 6, the fat was uniformly mixed at the ratio described in Table 13. POV of the mixed fat was 0. Thirty grams of the mixed fat were put into a 100-ml Erlenmeyer flask and stirred in a thermostatic shaking incubator (rotation number: 100 rpm, set at 60°C) with the flask opened, and sampling was carried out over time, to analyze POV (meq/kg). Additionally, as a method of adding ascorbic acid, an aqueous solution to which 16.7% by weight ascorbic acid-containing composition was dissolved was prepared, and 0.3 part by weight of the 16.7% by weight aqueous ascorbic acid solution, 0.1 part by weight of emulsifier (manufactured by RIKEN VITAMIN Co., Ltd., POEM PR-100) were added to 99.6 parts by weight of oil, and then stirred with a homomixer (TK ROBO MIX, manufactured by Tokusyu Kika Kogyo Co., Ltd.) at 10000 rpm × 5 minutes.

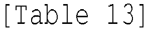

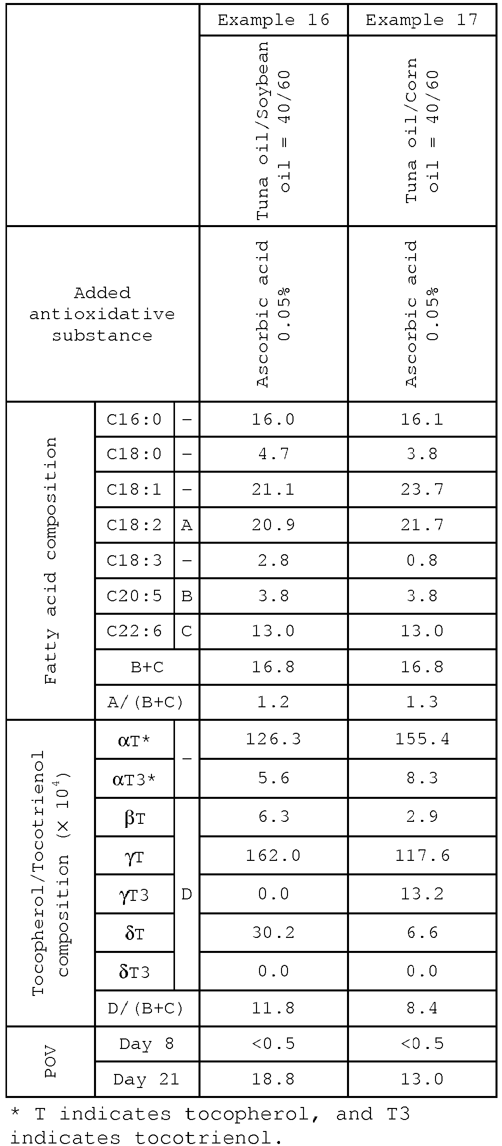

Oxidation stability was improved by adding ascorbic acid as is the case with addition of tea polyphenol, and a long-chain polyunsaturated fatty acid-containing fat having good oxidation stability of which POV value on day 8 is < 0.5 and the POV on day 21 was less than 20 was obtained.

### Industrial Applicability

According to the present invention, which is defined in scope by the claims, a long-chain polyunsaturated fatty acid-containing fat having improved oxidation stability and good flavor is obtained by a simple method, and a long-chain polyunsaturated fatty acid-containing fat wherein generation of reversion odor and unpleasant odor, and peroxide substance which adversely acts on health is suppressed for a long period of time, and having high health advantage is utilized for food, pharmaceutical composition, cosmetic, pet food, quasi drug and the like, by using the long-chain polyunsaturated fatty acid-containing fat of the present invention.

## Claims

1. A long-chain polyunsaturated fatty acid-containing fat comprising 1 to 5 parts by weight of linoleic acid and 0.001 part by weight to 0.05 part by weight of total amount of the following components (A), on the basis of 1 part by weight of the content of a long-chain polyunsaturated fatty acid having 5 or more double bonds in the constituent fatty acid composition, wherein
components (A): β-tocopherol, γ-tocopherol, δ-tocopherol, β-tocotrienol, γ-tocotrienol and δ-tocotrienol, and wherein
the content of the long-chain polyunsaturated fatty acid having 5 or more double bonds in the constituent fatty acid composition is 7 to 30% by weight.

2. The long-chain polyunsaturated fatty acid-containing fat according to claim 1, wherein the long-chain polyunsaturated fatty acid having 5 or more double bonds is DHA and/or EPA.

3. The long-chain polyunsaturated fatty acid-containing fat according to claim 1 or 2, further comprising an oxidation inhibitor.

4. The long-chain polyunsaturated fatty acid-containing fat according to claim 3, wherein the oxidation inhibitor is at least one agent selected from the group consisting of ascorbic acid, ascorbic acid derivative, tea extract, gallic acid, gallic acid derivative and lecithin.

5. The long-chain polyunsaturated fatty acid-containing fat according to any one of claims 1 to 4, which is in a state of liquid at 20°C.

6. The long-chain polyunsaturated fatty acid-containing fat according to claim 1, wherein a peroxide value after three days obtained from a preservation test of the long-chain polyunsaturated fatty acid-containing fat described below is 65 meq/kg or less:
(Method of preservation test of long-chain polyunsaturated fatty acid-containing fat)
• putting 30 g of uniformly mixed fat into a 100-ml Erlenmeyer flask;
• letting the Erlenmeyer flask opened to contact with air; and
• stirring the Erlenmeyer flask in a thermostatic shaking incubator (rotation number: 100 rpm, set at 60°C) with heating.

7. Use of the long-chain polyunsaturated fatty acid-containing fat according to any one of claims 1 to 5 in a food.

8. A method for suppressing increase in peroxide value of a long-chain polyunsaturated fatty acid-containing fat distributed in the state of liquid, comprising making a content of linoleic acid to 1 to 5 parts by weight and the total amount of the components (A) to 0.001 part by weight to 0.05 part by weight, on the basis of 1 part by weight of the content of the long-chain polyunsaturated fatty acid having 5 or more double bonds in the constituent fatty acid composition, wherein
components (A): β-tocopherol, γ-tocopherol, δ-tocopherol, β-tocotrienol, γ-tocotrienol and δ-tocotrienol, and wherein
the content of the long-chain polyunsaturated fatty acid having 5 or more double bonds in the constituent fatty acid composition is 7 to 30% by weight.

## Patentansprüche

1. Langkettige mehrfach ungesättigte Fettsäure enthaltendes Fett, das 1 bis 5 Gewichtsteile Linolsäure und 0,001 Gewichtsteile bis 0,05 Gewichtsteile der Gesamtmenge der folgenden Komponenten (A) umfasst, bezogen auf 1 Gewichtsteil des Gehalts einer langkettigen mehrfach ungesättigten Fettsäure mit 5 oder mehr Doppelbindungen in der konstituierenden Fettsäurezusammensetzung, wobei
Komponenten (A): β-Tocopherol, γ-Tocopherol, δ-Tocopherol, β-Tocotrienol, γ-Tocotrienol und δ-Tocotrienol, und wobei
der Gehalt der langkettigen mehrfach ungesättigten Fettsäure mit 5 oder mehr Doppelbindungen in der konstituierenden Fettsäurezusammensetzung 7 bis 30 Gew.-% beträgt.

2. Langkettige mehrfach ungesättigte Fettsäure enthaltendes Fett gemäß Anspruch 1, wobei es sich bei der langkettigen mehrfach ungesättigten Fettsäure mit 5 oder mehr Doppelbindungen um DHA und/oder EPA handelt.

3. Langkettige mehrfach ungesättigte Fettsäure enthaltendes Fett gemäß Anspruch 1 oder 2, das weiterhin ein Antioxidans umfasst.

4. Langkettige mehrfach ungesättigte Fettsäure enthaltendes Fett gemäß Anspruch 3, wobei das Antioxidans wenigstens ein Mittel ist, das aus der Gruppe ausgewählt ist, die aus Ascorbinsäure, einem Ascorbinsäure-Derivat, Teeextrakt, Gallussäure, einem Gallussäure-Derivat und Lecithin besteht.

5. Langkettige mehrfach ungesättigte Fettsäure enthaltendes Fett gemäß einem der Ansprüche 1 bis 4, das bei 20 °C im flüssigen Zustand vorliegt.

6. Langkettige mehrfach ungesättigte Fettsäure enthaltendes Fett gemäß Anspruch 1, wobei ein Peroxidwert nach drei Tagen, der aus einem im Folgenden beschriebenen Konservierungstest für das eine langkettige mehrfach ungesättigte Fettsäure enthaltende Fett erhalten wird, 65 mÄq/kg oder weniger beträgt:
(Methode des Konservierungstests für das eine langkettige mehrfach ungesättigte Fettsäure enthaltende Fett)
• Hineingeben von 30 g gleichmäßig gemischtem Fett in einen 100-ml-Erlenmeyerkolben;
• Offenlassen des Erlenmeyerkolbens für Kontakt mit Luft; und
• Rühren des Erlenmeyerkolbens in einem thermostatischen Schüttelinkubator (Drehzahl: 100 U/min, eingestellt auf 60 °C) unter Erhitzen.

7. Verwendung des eine langkettige mehrfach ungesättigte Fettsäure enthaltenden Fetts gemäß einem der Ansprüche 1 bis 5 in einem Lebensmittel.

8. Verfahren zum Unterdrücken der Erhöhung des Peroxidwerts eines eine langkettige mehrfach ungesättigte Fettsäure enthaltenden Fetts, das im flüssigen Zustand verteilt ist, umfassend das Einstellen des Gehalts an Linolsäure auf 1 bis 5 Gewichtsteile und der Gesamtmenge der Komponenten (A) auf 0,001 Gewichtsteile bis 0,05 Gewichtsteile, bezogen auf 1 Gewichtsteil des Gehalts der langkettigen mehrfach ungesättigten Fettsäure mit 5 oder mehr Doppelbindungen in der konstituierenden Fettsäurezusammensetzung, wobei
Komponenten (A): β-Tocopherol, γ-Tocopherol, δ-Tocopherol, β-Tocotrienol, γ-Tocotrienol und δ-Tocotrienol, und wobei
der Gehalt der langkettigen mehrfach ungesättigten Fettsäure mit 5 oder mehr Doppelbindungen in der konstituierenden Fettsäurezusammensetzung 7 bis 30 Gew.-% beträgt.

## Revendications

1. Graisse contenant un acide gras polyinsaturé à chaîne longue comprenant de 1 à 5 parties en masse d'acide linoléique et de 0,001 partie en masse à 0,05 partie en masse de quantité totale des constituants (A) suivants, sur la base de 1 partie en masse de la teneur d'un acide gras polyinsaturé à chaîne longue ayant 5 doubles liaisons ou plus dans la composition d'acide gras constituante, dans laquelle
constituants (A) : β-tocophérol, γ-tocophérol, δ-tocophérol, β-tocotriénol, γ-tocotriénol et δ-tocotriénol, et dans laquelle
la teneur de l'acide gras polyinsaturé à chaîne longue ayant 5 doubles liaisons ou plus dans la composition d'acide gras constituante est de 7 à 30 % en masse.

2. Graisse contenant un acide gras polyinsaturé à chaîne longue selon la revendication 1, dans laquelle l'acide gras polyinsaturé à chaîne longue ayant 5 doubles liaisons ou plus est DHA et/ou EPA.

3. Graisse contenant un acide gras polyinsaturé à chaîne longue selon la revendication 1 ou 2, comprenant de plus un inhibiteur d'oxydation.

4. Graisse contenant un acide gras polyinsaturé à chaîne longue selon la revendication 3, dans laquelle l'inhibiteur d'oxydation est au moins un agent choisi dans le groupe consistant en acide ascorbique, dérivé d'acide ascorbique, extrait de thé, acide gallique, dérivé d'acide gallique et lécithine.

5. Graisse contenant un acide gras polyinsaturé à chaîne longue selon l'une quelconque des revendications 1 à 4, qui est dans un état de liquide à 20°C.

6. Graisse contenant un acide gras polyinsaturé à chaîne longue selon la revendication 1, dans laquelle une valeur peroxyde après trois jours obtenue à partir d'un test de préservation de la graisse contenant un acide gras polyinsaturé à chaîne longue décrit ci-dessous est de 65 meq/kg ou inférieure :
(méthode de test de préservation de graisse contenant un acide gras polyinsaturé à chaîne longue)
• dépose de 30 g de graisse uniformément mixée dans une fiole Erlenmeyer de 100 ml ;
• laisser la fiole Erlenmeyer ouverte pour être en contact avec l'air ; et
• agitation de la fiole Erlenmeyer dans un incubateur à secousses thermostatique (nombre de rotations : 100 tr/min, réglé à 60°C) avec chauffage.

7. Utilisation de la graisse contenant un acide gras polyinsaturé à chaîne longue selon l'une quelconque des revendications 1 à 5 dans un aliment.

8. Procédé de suppression d'augmentation de valeur peroxyde d'une graisse contenant un acide gras polyinsaturé à chaîne longue distribuée dans l'état de liquide, comprenant la constitution d'une teneur d'acide linoléique à de 1 à 5 parties en masse et de la quantité totale des constituants (A) à de 0,001 partie en masse à 0,05 partie en masse, sur la base de 1 partie en masse de la teneur de l'acide gras polyinsaturé à chaîne longue ayant 5 doubles liaisons ou plus dans la composition d'acide gras constituante, dans lequel
constituants (A) : β-tocophérol, γ-tocophérol, δ-tocophérol, β-tocotriénol, γ-tocotriénol et δ-tocotriénol, et dans lequel
la teneur de l'acide gras polyinsaturé à chaîne longue ayant 5 doubles liaisons ou plus dans la composition d'acide gras constituante est de 7 à 30 % en masse.
